**Europäisches Patentamt**

(19) **European Patent Office**

**Office européen des brevets**

(11) Numéro de publication: **0 155 868**
**B1**

(12) **FASCICULE DE BREVET EUROPÉEN**

(45) Date de publication du fascicule du brevet:
**12.08.87**

(51) Int. Cl.⁴: **B 01 J 23/80,** C 07 C 29/15,
C 01 B 3/22

(21) Numéro de dépôt: **85400305.0**

(22) Date de dépôt: **20.02.85**

(54) **Procédé de fabrication de catalyseurs contenant du cuivre, du zinc, de l'aluminium et au moins un métal du groupe formé par les terres rares et le zirconium et utilisation des catalyseurs obtenus pour les réactions mettant en jeu un gaz de synthèse.**

(30) Priorité: **02.03.84 FR 8403325**

(43) Date de publication de la demande:
**25.09.85 Bulletin 85/39**

(45) Mention de la délivrance du brevet:
**12.08.87 Bulletin 87/33**

(84) Etats contractants désignés:
**BE DE GB IT NL SE**

(56) Documents cité:
**EP-A-0 034 338**
**FR-A-2 181 002**
**GB-A-1 131 631**
**US-A-4 257 920**
**US-A-4 375 424**

(73) Titulaire: **INSTITUT FRANCAIS DU PETROLE, 4, Avenue de Bois- Préau, F-92506 Rueil- Malmaison Cédex (FR)**

(72) Inventeur: **Courty, Philippe, 91, rue Condorcet, F-78800 Houilles (FR)**
Inventeur: **Durand, Daniel, 18, rue Michelet, F-92500 Rueil- Malmaison (FR)**
Inventeur: **Travers, Christine, 15 ter, rue Beaumarchais, F-92500 Rueil- Malmaison (FR)**
Inventeur: **Chaumette, Patrick, 10, rue Massena, F-92500 Rueil- Malmaison (FR)**
Inventeur: **Forestière, Alain, Le Prévert Bt B, Route de Lyon, F-69390 Vernaison (FR)**

**0 155 868**

## Description

La présente invention concerne un procédé de préparation d'un catalyseur de composition essentiellement homogène, particulièrement actif, stable et sélectif, utilisable dans des procédés mettant en jeu une réaction équilibrée des oxydes de carbone (CO, $CO_2$) avec l'hydrogène, en particulier la production de méthanol à partir de gaz de synthèse et les réactions de décomposition des alcools, et en particulier du méthanol en un mélange contenant des oxydes de carbone et de l'hydrogène.

Ces catalyseurs contiennent au moins quatre métaux: cuivre, aluminium, zinc, et au moins un métal du groupe formé par les terres rares de numéros atomiques 57 à 71 inclus et le zirconium. Ils peuvent éventuellement contenir, en outre, de 0,01 à 1% d'au moins un métal choisi dans le groupe formé par le palladium, le rhénium, le platine. Ils peuvent également contenir, en outre, de 0,5 à 5% d'argent.

Les catalyseurs à base d'oxydes de cuivre et de zinc sont connus depuis de nombreuses années; ils ont été décrits dés 1933 par DODGE (US 1 908 696). Dans les brevets US 3 388 972, 3 546 140, 3 790 505 la société américaine C.C.I. décrit l'utilisation de compositions ternaires Cu Zn Al pour la conversion basse température du CO, et pour la synthése du méthanol. Dans le brevet français 2 113 467, la société SHELL préconise l'emploi de catalyseurs Cu Zn Dy pour la synthése du méthanol; Dy, ou didyme représentent le mélange d'au moins deux oxydes des métaux des terres rares de numéros atomiques 57 à 71 inclus. Cette société montre que les meilleurs résultats correspondent à des catalyseurs contenant plusieurs oxydes des métaux des terres rares; l'emploi d'aluminium n'est pas suggéré.

Divers modes de préparation des catalyseurs Cu Zn Al sont décrits, notamment dans les brevets US 3 923 694 (I.C.I.) et US 4 279 781 (United Catalysts).

L'adjonction de métaux précieux, sous forme de chlorures, a été revendiquée entre autres, dans le brevet américain US 4 257 920 qui concerne des catalyseurs Cu Al Zn préparés par mélange de leurs oxydes et/ou carbonates en présence d'un ciment alumineux, le catalyseur contenant en outre au moins un oxyde des métaux des terres rares, de numéros atomiques 57 à 71 inclus.

La présente invention concerne un procédé de préparation des dits catalyseurs; on a en effet découvert que des catalyseurs particulièrement actifs, sélectifs et stables pouvaient être obtenus par la succession d'étapes suivantes:

Etape a:
Coprécipitation par un réactif alcalin d'un précurseur,
à un pH fixé à une valeur comprise entre 6,3 et 7,3 environ et de préférence à une valeur comprise entre 6,6 et 7,0 unités pH environ contenant les métaux cuivre, aluminium, zinc ainsi qu'éventuellement de l'argent et/ou du palladium et/ou du rhénium.

Etape b:
Lavage de façon à ramener la teneur en métal alcalin (apporté par le réactif de coprécipitation) du précipité lavé à moins de 0,06 % environ en poids et préférentiellement moins de 0,04% environ par rapport aux métaux.

Etape c:
Coprécipitation par un composé d'ammonium (hydroxyde, carbonate, hydroxycarbonate) d'un composé de terre rare et/ou de zirconium, ou d'un mélange de composé par exemple à partir de nitrate du même métal.

Etape d:
Lavage de façon à amener la teneur en azote du précipité lavé à moins de 3% en poids environ et préférentiellement moins de 1% environ par rapport aux métaux.

Etape e:
Mélange des deux précipités de manière à obtenir une dispersion intime, à l'échelle de 0,01-0,1 µ (micron) du précipité de terre rare et/ou du zirconium et du précurseur contenant les métaux Cu, Al, Zn ainsi qu'éventuellement Pd et/ou Ag et/ou Re.

Etape f:
Séchage, puis activation thermique.

Au moins un métal M du groupe constitué par l'ensemble palladium, rhénium, platine et argent peut aussi éventuellement être déposé par imprégnation du catalyseur activé. On a découvert que les ions chlorure et sulfate ont un effet négatif sur leur activité et que des performances améliorées sont obtenues avec d'autres sels. On utilisera par exemple les acétylacétonates, les nitrates, les complexes amminés; le rhénium est de préférence sous forme d'acide perrhénique ou de ses sels.

Les catalyseurs selon la présente invention contiennent les métaux précités dans les proportions suivantes:
Cuivre de 20 à 80% et préférentiellement de 45 à 70%
Zinc de 5 à 50% et préférentiellement de 15 à 35%
Aluminium de 2 à 30% et préférentiellement de 4 à 20%
Métaux des
Terres rares,
et/ou Zirconium de 2 à 20% et préférentiellement de 5 à 18%
Ils peuvent éventuellement contenir, en outre, jusqu'à 10% en poids, par exemple et préférentiellement jusqu'à 5% en poids de métaux d'au moins un métal M choisi dans le groupe formé par le palladium, le rhénium, le platine et l'argent.

Lorsque les catalyseurs contiennent un ou plusieurs métaux du groupe formé par le palladium, le rhénium et le platine, la teneur de ces métaux exprimée en poids est de façon la plus préférée comprise entre 0,01 et 1%

2

et s'ils contiennent de l'argent la teneur en argent est de façon la plus préférée comprise entre 0,5 et 5%.

Les métaux Cu, Zn, Al ainsi que séparément ou simultanément les terres rares, le zirconium, le palladium, l'argent sont mis en oeuvre sous forme de composés solubles et de préférence solubles en milieu acide, bien que les complexes amminés (solubles en milieu ammoniacal) du cuivre, du zinc, du palladium puissent éventuellement être utilisés en addition au réactif alcalin et/ou ammoniacal de coprécipitation.

On utilisera par exemple les oxydes solubles (par exemple $Re_2O_7$), les hydroxydes, les carbonates, les hydroxycarbonates solubles en milieu acide (par exemple $Cu\ CO_3 - Cu(OH)_2$, $Zn\ CO_3$, $Zn(OH)_2$) les nitrates, oxalates, tartrates, citrates, acétates, acétylacétonates ou encore des combinaisons anioniques telles que aluminate, ou perrhénate. Les nitrates sont les sels solubles qui sont le plus souvent mis en oeuvre.

Pour la préparation de ces masses catalytiques, il est essentiel d'utiliser des techniques de préparation permettant d'obtenir un produit de composition aussi homogène que possible, et évitant la ségrégation des différents éléments lors des différentes étapes unitaires de la préparation.

Un mode de préparation consiste à préparer, par au moins une réaction de coprécipitation, un précurseur hydraté, contenant les métaux Cu Zn Al et éventuellement Pd et/ou Ag; la réaction de coprécipitation consiste à mettre en présence dans des conditions opératoires définies plus loin la solution de sels solubles des métaux Cu Zn Al, éventuellement Pd et/ou Ag avec une solution de carbonate et/ou hydrogénocarbonate et/ou d'hydroxyde de sodium et/ou de potassium de façon à obtenir un coprécipité qui, après lavage ultérieur, constitue le précurseur hydroxycarbonate hydraté.

On entend par hydroxycarbonate un composé hydraté dans lequel les groupements hydroxyles sont substitués au moins en partie par des ions carbonates ; les dits ions carbonates peuvent être introduits lors de la coprécipitation (par exemple précipitation par les carbonates) ou encore provenir d'une réaction avec le $CO_2$ dissous et/ou le $CO_2$ atmosphérique qui peuvent interagir avec le précipité lors des étapes de filtration, lavage et séchage.

Toutes les techniques et appareillages qui ont été décrits dans l'art antérieur peuvent être utilisés ou appliqués à la réalisation de l'invention: on peut par exemple ajouter la solution de sels des métaux Cu Zn Al et éventuellement Pd et/ou Ag dans la solution alcaline ou l'inverse. De préférence, on ajoutera les deux solutions, de façon simultanée, et en régulant leurs débits par le pH mesuré dans la zone réactionnelle, dans un réacteur comportant un systéme d'agitation efficace. D'une façon préférée, les deux solutions seront mises en contact dans la zone de turbulence maximum délimitée par le volume enveloppant l'appareillage d'agitation à l'intérieur du volume réactionnel.

Le temps de séjour moyen, exprimé en minutes est défini comme le rapport du débit volumique total (litres/minute) des solutions injectées dans le réacteur, au volume du dit réacteur, exprimé en litres. Dans un réacteur opérant en "batch", où le temps de séjour est compris entre 30 et 300 minutes environ et de préférence entre 60 et 180 minutes environ les réactifs sont injectés en continu, sans récupération parallèle du produit de réaction, ce produit de réaction restant en présence de réactifs injectés en continu. Ce type de réacteur dont le volume (compte-tenu des spécifications de concentration des solutions utilisées et des quantités de catalyseur à préparer) varie environ de 1 litre à environ 1000 litres ou plus, opère à concentrations variables, les autres conditions opératoires restant constantes au cours de la précipitation elle-même. Ce mode de réaction est bien adapté à la préparation de composés cristallisés.

Un mode de réalisation de l'invention consiste à faire réagir à une température d'au moins 60°C environ et de préférence d'au moins 70°C environ, une solution de sels des métaux Cu Zn Al, éventuellement Pd et/ou Ag, de concentration globale au plus égale à 2 at.g environ de métaux par litre, par exemple entre 0,3 et 1,8 at.g environ de métaux par litre, avec une solution de carbonate et/ou d'hydrogénocarbonate et/ou d'hydroxyde de sodium et/ou de potassium et/ou d'ammonium de concentration globale au plus égale à 4 at.g environ (par exemple comprise entre 0,6 et 3,6 at.g environ de métaux alcalins par litre, la réaction de coprécipitation (étape a) étant menée à un pH fixé à une valeur comprise entre 6,3 et 7,3 environ, et de préférence entre 6,6 et 7,0 unité pH environ, et le temps de résidence dans le milieu réactionnel étant d'au moins 60 minutes environ; on obtient de la sorte un précipité contenant un hydroxycarbonate mixte hydraté, cristallisé au moins en partie dans une ou plusieurs structures. Lorsque le cation alcalin est le sodium, la solution alcaline peut contenir éventuellement, en outre, le rhénium, au moins partiellement, sous forme d'anion perrhénate ($ReO_4$). L'ion carbonate provient indifféremment de l'une ou l'autre solution ou provient de l'interaction du $CO_2$ dissous et du $CO_2$ atmosphérique avec le précipité formé.

Le ou les composés cristallisés peuvent éventuellement être mûri par exemple entre 50 et 80°C environ à pression atmosphérique pendant 15 minutes à 5 heures, en présence des eaux mères ou encore des eaux de lavage. Au cours de cette opération de mûrissement, on note en général une augmentation du pH, généralement au plus égale à 1,5 unité pH, par rapport au pH de précipitation. On observe que, d'une façon inattendue ce traitement de mûrissement améliore la cristallinité et/ou augmente la taille des cristallites du précurseur hydraté cristallisé.

L'opération de mûrissement peut être menée dans le même réacteur, après arrêt de l'injection des réactifs. On peut également, dans le cas de la précipitation continue, recueillir le précipité obtenu en conditions stationnaires (température, concentrations, pH, vitesse d'introduction des réactifs) et le mûrir, après lavage éventuel, dans un autre réacteur.

De préférence, pour la préparation du précipité cristallisé, la température réactionnelle sera d'au moins 70°C environ, la concentration de la solution de sels des métaux Cu Zn Al éventuellement Pd, Ag, Re, sera comprise entre 0,6 et 1,5 at.g environ de métaux par litre et celle des composés des métaux alcalins entre 1,2 et 3,0 at.g environ de métaux alcalins par litre, et le temps de réaction sera d'au moins 120 minutes environ. La solution des métaux alcalins étant de préférence une solution de carbonate.

Après précipitation et mûrissement éventuel dans les eaux mères, le précipité cristallisé est lavé (étape b) de façon à réduire sa teneur en alcalin (exprimée en poids d'alcalins par rapport au poids total des métaux) à moins de 0,06% poids environ, cette teneur en alcalin résiduel est en général d'une manière préférée inferieure à 0,04% poids, puis mûri éventuellement dans les eaux de lavage.

Le composé comprenant au moins un métal Ln du groupe des terres rares, de numéros 57 à 71 inclus, et/ou le zirconium est préparé par réaction de coprécipitation (étape c), dans les conditions opératoires précitées, à partir d'au moins un des composés solubles précités, l'agent coprécipitant étant au moins un composé du cation ammonium (hydroxyde, carbonate, hydrogénocarbonate). Seul le pH de coprécipitation peut varier dans un intervalle plus large que celui précité; un pH de coprécipitation compris entre 6 et 8 unités pH environ conduit à des résultats satisfaisants.

De même après coprécipitation, le composé hydraté contenant au moins un métal Ln et/ou zirconium peut éventuellement être mûri dans les conditions opératoires décrites ci-avant.

Après précipitation et mûrissement éventuel dans les eaux mères, le précipité contenant au moins un métal Ln et/ou le zirconium est lavé (étape d) de façon à réduire sa teneur en azote ($NH_4^+$ et, éventuellement $NO_3^-$) à une valeur, rapporté au poids total de métaux inférieure à 3 % et préférentiellement inférieure à 1 % poids.

Les deux coprécipités lavés obtenus comme il vient d'être dit sont ensuite mélangés (étape e) dans un appareillage permettant d'obtenir une dispersion aussi homogène que possible des deux produits l'un dans l'autre. La dispersion peut être mesurée à la microsonde de Castaing ou encore par microanalyse X au microscope à balayage (STEM). Les meilleurs résultats sont obtenus pour une dispersion sensiblement homogène à l'échelle 0,01-0,1 (micron).

La dispersion sensiblement homogène des deux coprécipités est obtenue, par utilisation de leurs propriétés thixotropes, en soumettant le mélange à des forces de cisaillement suffisamment importantes, appliquées au produit par l'intermédiaire de pales tournantes, de disques, de cylindres ou encore par passage au travers d'orifices, par exemple dans les mélangeurs Werner, Cowles, Waring, Hockmeyer. Rousselle, et dans certains mélangeurs à galets. Après arrêt du malaxage cisaillant, aucune décantation et/ou ségrégation ne doit être observée.

Le séchage du produit mixte homogénéisé peut être réalisé par tout procédé connu: on peut l'effectuer par exemple par atomisation (spray-drying). On obtient un produit sensiblement homogène, en poudre calibrée contenant d'environ 60 à environ 80 % en poids d'équivalent d'oxydes. On peut également sécher le produit en étuve, par exemple entre environ 50 et environ 150° C, sous balayage d'air, de façon à ramener si nécessaire la teneur en oxydes potentiels à environ 60 à 80 % en poids. Il est recommandé d'éviter la stagnation du précipité en présence de pressions partielles de vapeur d'eau proches de la pression de vapeur saturante à la température de séchage considérée; de tels traitements peuvent avoir pour conséquence une déshydratation partielle du précipité avec cristallisation d'oxyde cuivrique en gros cristallite.

L'activation thermique consiste à traiter le précipité séché, à une température d'environ 250 à environ 500° C et de préférence entre environ 300 et environ 380° C pendant une durée suffisante, par exemple pendant au moins 0,5 heure pour obtenir catalyseur activé sensiblement homogène ne renfermant pas plus de 12% poids de matières volatiles (le taux de matières volatiles est mesuré par exemple par activation en présence d'air d'un poids donné de produit, placé dans une nacelle et calciné à 500-600°C pendant 4 heures).

L'activation thermique peut être menée par exemple en présence d'un gaz inerte contenant de 0 à 50% d'oxygéne.

Ces opérations de séchage et d'activation thermique (étape f) peuvent aussi être combinées par utilisation de techniques de grillage-éclair ou de calcination par atomisation (spray-calcination), le produit étant alors pulvérisé dans un courant de gaz de combustion.

Le catalyseur activé thermiquement à une température comprise entre environ 250°C et environ 500°C puis éventuellement broyé peut ensuite être éventuellement mis en contact avec une solution aqueuse ou organique d'au moins un métal M choisi dans le groupe formé par le palladium, le platine, le rhénium et/ou l'argent, de façon à disperser d'une manière essentiellement uniforme le dit métal, et à obtenir après séchage et activation thermique comme ci-avant un catalyseur où le dit métal est bien dispersé (la dispersion peut par exemple se mesurer par chimisorption de gaz réactifs CO, $H_2$, sur le dit métal). A l'exception des halogénures et des sulfates, tous les sels solubles, par exemple nitrates, acétylacétonates, ainsi que les complexes par exemple nitrosamminés, amminés, carbonylés sont utilisables.

La mise en forme du catalyseur est réalisée par tout procédé connu, parexemple par traitement du précipité humide après dépôts des métaux additionnels, du précipité séché, du précipité activé thermiquement; les opérations de mise en forme par extrusion, dragéification, coagulation en gouttes sont utilisables; on peut aussi broyer le produit homogène activé thermiquement, par exemple en-dessous de 0,5 mm, le mélanger à raison de 0,5-5% de son poids avec au moins un composé adjuvant de pastillage choisi dans le groupe formé par le graphite, l'acide stéarique, les stéarates, et éventuellement un adjuvant de porosité choisi parmi la cellulose et les poudres d'origine végétale en contenant, les carbonates d'ammonium, les fibres textiles combustibles, le naphtalène, puis enfin le pastiller en cylindres pleins de diamètres 3-6 mm ou en cylindres toriques de diamètres extérieur 3-6 mm et intérieur 1 à 4 mm et de hauteur 2 à 6 mm.

Le catalyseur mis en forme par pastillage subira éventuellement une ultime activation thermique dans les conditions opératoires précitées.

4

**0 155 868**

Le catalyseur activé thermiquement se compose essentiellement d'une association sensiblement homogène d'oxydes. Dans cette association homogène d'oxydes, les métaux et notamment le cuivre, le zinc, l'aluminium et les autres éléments métalliques additionnels sont répartis à l'échelle de 0,01 - 0,1 $\mu$m de manière homogène. La surface spécifique desdits catalyseurs varie entre environ 50 et environ 150 $m^2g^{-1}$.

Les conditions de mise en oeuvre des dits catalyseurs pour la fabrication du méthanol sont habituellement les suivantes: le catalyseur, chargé au réacteur, est tout d'abord préréduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/composé réducteur + gaz inerte étant de 0,001: 1 à 1: 1.

La température de réduction varie généralement de 100 à 300°C environ mais de préférence entre 140 et 260°C environ, la pression totale est habituellement comprise entre 0,1 et 10 MPa environ et de préférence entre 0,1 et 6 MPa environ; la vitesse volumétrique horaire est habituellement comprise entre $10^2$ et $4.10^4$ heure$^{-1}$ environ et de préférence entre $5.10^2$ et $10^4$ heure$^{-1}$ environ. (Température et pression normale,TPN).

La réduction est d'abord menée, par exemple entre environ 140 et environ 160°C, en présence du mélange réducteur précité et avec un rapport molaire gaz réducteur/gaz réducteur + gaz inerte compris entre-0,001 et 0,1 environ et préférentiellement entre 0,005 et 0,05 environ, pendant un temps suffisant pour que les concentrations en gaz réducteur soient les mêmes à l'entrée et à la sortie du réacteur (ce qui prouve que la première étape de réduction est terminée), il peut être avantageux dans une deuxième étape d'augmenter la température ainsi qu'éventuellement la concentration en gaz réducteur, et de poursuivre la réduction dans des conditions thermiques plus sévères.

La température de réduction varie alors entre environ 160°C et environ 240°C, le rapport molaire gaz réducteur/gaz réducteur + gaz inerte est alors compris entre 0,01 et 1 environ, et préférentiellement entre 0,05 et 1 environ, les pressions et vitesse volumétrique horaire restant à l'intérieur des fourchettes précitées.

La préréduction du catalyseur sera préférentiellement menée en phase liquide si, par la suite, la réaction de synthése du méthanol se déroule en phase liquide.

La réaction proprement dite de synthèse du méthanol est réalisée dans les conditions opératoires suivantes: la pression est habituellement comprise entre 2 et 15 MPa environ et de préférence entre 4 et 15 MPa environ, le rapport molaire $H_2/2CO + 3CO_2$ est avantageusement compris entre 0,4 et 10 environ, mais de préférence entre 0,5 et 4 environ, lorsque la réaction est menée en phase gazeuse, et comprise de préférence entre 0,5 et 1,5 environ lorsque la réaction est menée en phase liquide, et la température est comprise entre 200 et 300°C environ, mais de préférence entre 220 et 270°C environ.

La vitesse volumique horaire (exprimée en volume à température et pression normale de mélange gazeux par volume de catalyseur et par heure) est usuellement comprise entre 1500 et 60.000 h$^{-1}$ environ et de préférence entre 2000 et 20.000 h$^{-1}$ environ.

Le catalyseur peut être utilisé en poudre fine calibrée (10-700 $\mu$m environ) ou en particules de diamètre équivalent 2 à 10 mm environ, en présence d'une phase gazeuse, ou d'une phase liquide (dans les conditions opératoires) et d'une phase gazeuse. La phase liquide peut être constituée par un ou plusieurs hydrocarbures ayant au moins 5 et de préférence au moins 10 atomes de carbone.

Dans ce mode de mise en oeuvre, il est préférable que les vitesses superficielles du gaz et du liquide, dans les conditions de température et de pression du procédé, soient d'au moins 1,5 cm/sec. environ et de préférence d'au moins 3 cm/sec. environ. Par vitesse superficielle on entend le rapport du débit volumique à la section du réacteur considéré vide de catalyseur.

Les conditions de mise en oeuvre des dits catalyseurs pour la décomposition des alcools de $C_1$ à $C_5$ et en particulier du méthanol sont habituellement les suivantes: le catalyseur, chargé au réacteur, est tout d'abord préréduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/composé réducteur + gaz inerte étant de 0,001: 1 à 1: 1 environ.

La température de réduction varie généralement de 100 à 300°C environ mais de préférence entre 140 et 260°C environ, la pression totale est habituellement ccmprise entre 0,1 et 10 MPa environ et de préférence entre 0,1 et 6 MPa environ; la vitesse volumétrique horaire est habituellement comprise entre $10^2$ et $4.10^4$ heure$^{-1}$ environ et de préférence entre $5.10^2$ et $10^4$ heure$^{-1}$ environ. (Température et pression normale TPN).

La réduction est d'abord menée, par exemple entre environ 140 et environ 160°C, en présence du mélange réducteur précité et avec un rapport molaire gaz réducteur/gaz réducteur + gaz inerte compris entre 0,001 et 0,1 environ et préférentiellement entre 0,005 et 0,05 environ, pendant un temps suffisant pour que les concentrations en gaz réducteur soient les mêmes à l'entrée et à la sortie du réacteur (ce qui prouve que la première étape de réduction est terminée),il peut être avantageux dans une deuxième étape d'augmenter la température ainsi qu'éventuellement la concentration en gaz réducteur, et de poursuivre la réduction dans des conditions thermiques plus sévères.

La température de réduction varie alors entre environ 160 et environ 240°C, le rapport molaire gaz réducteur/gaz réducteur + gaz inerte est alors compris entre 0,01 et 1 environ, et préférentiellement entre 0,05 et 1 environ, les pressions et vitesse volumétrique horaire restant à l'intérieur des fourchettes précitées.

La réaction proprement dite de décomposition est réalisée dans les conditions opératoires suivantes: la pression est habituellement comprise entre 1 et 10 MPa environ et de préférence entre 2 et 5 MPa environ, la température est comprise entre 200 et 400°C environ, mais de préférence entre 220 et 320°C environ.

La vitesse volumique horaire de la charge (exprimée en litre de charge par litre de catalyseur et par heure)

5

est usuellement comprise entre 0,1 et 5 h$^{-1}$ environ et de préférence entre 0,5 et 3 h$^{-1}$ environ.

Les exemples qui font suite décrivent différents aspects de l'invention, sans en limiter la portée.

Exemples 1 à 18.

On décrit tout d'abord la préparation des catalyseurs A à H dont les caractéristiques figurent dans le tableau II; les modalités de préparation sont résumées dans le tableau III. Les préparations des catalyseurs A à C et F à H sont décrites de façon détaillée ci-après et résumées dans le tableau III pour les autres catalyseurs. Les catalyseurs A, $A_1$, $A_2$, D, E, $E_1$, F, G, $K_1$ sont des catalyseurs préparés à titre de comparaison.

CATALYSEUR A: (comparaison)

- On dissout dans 1,5 l d'eau, 241,6 g de nitrate cuivrique trihydraté (1 at.g Cu), 150 g de nitrate d'aluminium nonahydraté (0,4-at.g Al) et 89,25 g de nitrate de zinc hexahydraté (0,3 at.g Zn). La solution (solution A, 1,133 at.g de métaux/l) est portée à 80° C.

On dissout séparément 234,25 g de carbonate disodique dans 1,5 l d'eau, cette solution B (2,95 at.g Na/l), est portée à 80°C.

- Dans un réacteur chauffé, de capacité 10 l, contenant 3 l d'eau portée à 80°C on ajoute simultanément les solutions A et B en 2 heures, les débits étant régulés par le pH, ce dernier variant entre 6,75 et 6,95. Le précipité obtenu est mûri à 80°C dans ses eaux mères pendant 30 minutes avant d'être lavé 3 fois par 10 litres d'eau. Ce précipité cristallisé, humide contient environ 22% en poids d'oxydes des métaux non alcalins et 132 ppm poids de sodium par rapport aux oxydes. Il est ensuite séché en étuve ventilée pendant 16 heures à 40°C puis 4 heures à 90°C (il contient alors 72% en poids d'oxydes), puis activé 4 heures à 350°C sous air. La teneur en matières volatiles résiduelles est alors de 5%. La poudre obtenue par broyage en-dessous de 0,5 mm est mélangée avec 2% en poids de graphite, pastillée en cylindre de 4 mm de diamètre et 3,5 mm de longueur, puis activée 3 heures à 350°C sous air. On obtient environ 130 g de catalyseur de surface spécifique 65 m$^2$/g.

CATALYSEUR $A_1$: (comparaison)

La préparation d'un catalyseur A est effectuée selon la méthode décrite pour le catalyseur A à l'exception du fait que la solution A contient en plus 43,30 g de nitrate de lanthane hexahydraté (0,1 at.g La) soit au total 1,2 at.g de métaux par litre. La solution B est obtenue par dissolution de 238,5 g de carbonate disodique dans 1,5 l d'eau (3 at.g Na/l).

CATALYSEUR $A_2$ (comparaison)

Le mode de preparation du catalyseur $A_2$ est identique à celui décrit pour le catalyseur $A_1$ à l'exception du fait que l'on augmente considérablement les quantités d'eau de lavage pour abaisser la teneur en sodium dans le catalyseur final à 600 ppm en poids.

CATALYSEUR B :

- On dissout dans 1,5 l d'eau, 241,6 g de nitrate de cuivre trihydraté (1 at.g Cu), 56,25 g de nitrate d'aluminium nonahydraté (0,15 at.g Al), 89,25 g de nitrate de zinc héxahydraté (0,3 at.g Zn). La solution (solution A, 0,966 at.g de métaux/l) est portée à 80°C.

- On dissout séparément 199 g de carbonate disodique dans 1,5 l d'eau, cette solution B (2,50 at.g Na/l) est portée à 80°C.

- Dans un réacteur chauffé, de capacité 10 l, contenant 3 l d'eau portée à 80°C, on ajoute simultanément les solutions A et B en 2 heures, les débits étant régulés par le pH, ce dernier variant entre 6,75 et 6,85. Le précipité (P) obtenu est mûri à 80°C dans les eaux mères pendant 30 minutes. Il est ensuite lavé 3 fois par 10 l d'eau. Sa teneur en sodium est de 180 ppm en poids. Le tableau I donne les indices de Miller de la phase rhomboédrique, type hydroxycarbonate du précipité cristallisé obtenu.

- Dans un second réacteur, de capacité 10 l, contenant 3 l d'eau portée à 80°C, on ajoute simultanément deux solutions C et D.

La première, solution (C), contient 65,15 g de nitrate de cérium hexahydraté dissous dans 1 litre d'eau (0,15 at.g/l); elle est portée à 80°C.

La seconde, solution (D), est une solution 0,5 molaire d'ammoniaque.

Les débits de chaque solution sont régulés par le pH, ce dernier est maintenu à environ 7, la température de précipitation est fixée à 80°C. Le précipité d'hydroxyde de cérium obtenu est lavé 3 fois par 10 l d'eau. Sa teneur en azote est alors inférieure à 1% en poids par rapport au poids d'oxyde potentiel.

Dans un réacteur de 10 l contenant 6 l d'eau à 20°C, on mélange le précipité (P) et l'hydroxyde de cérium obtenus ci-dessus. Un brassage efficace de cette suspension au moyen d'une turbine permet d'obtenir une dispersion intime à l'échelle de 0,01 à 0,1 micron de tous les constituants de de nouveau précurseur. Les étapes de séchage, calcination et de mise en forme sont identiques à celles décrites pour le catalyseur A.

CATALYSEUR C:

Les méthodes de préparation du précipité P (mélange contenant des hydroxycarbonates de cuivre, aluminium et zinc) et d'un hydroxyde de terres rares sont identiques à celle utilisées pour la préparation du catalyseur B. Seule, leur méthode de mélange est différente. En effet les deux précipités humides, séparés de leur derniére eau de lavage, sont mélangés à l'état humide dans un malaxeur. Après un temps de malaxage d'environ 1/2 heure permettant d'obtenir une bonne dispersion de tous les éléments (à l'échelle de 0,1 micron) le produit obtenu subit comme dans les exemples ci-dessus les mêmes étapes de séchage, activation et mise en forme.

CATALYSEUR D: (comparaison)

On mélange mécaniquement et à sec le produit séché (contenant des hydrocarbonates de Cu, Al et Zn) obtenu suivant la préparation du catalyseur A avec un carbonate de terre rare ($La_2(CO_3)_3, sH_2O$). La poudre

**0 155 868**

homogène (à l'échelle du micron) ainsi obtenue est ensuite activée sous air à 350°C pendant 4 heures puis mise en forme par la même méthode que celle employée pour le catalyseur A.

CATALYSEUR E: (comparaison)

On mélange mécaniquement et à sec le produit activé sous air à 350°C obtenu suivant la méthode de préparation du catalyseur A (oxyde mixte de Cu, Zn et Al) avec un oxyde de terre rare ($La_2O_3$). Après homogénéisation à l'échelle du micron des différents oxydes, la poudre est mise en forme puis réactivée sous air à 350°C.

CATALYSEUR $E_1$ (Comparaison)

Le mode de préparation du catalyseur $E_1$ est identique à celui décrit pour le catalyseur A à l'exception du fait que l'on réalise, sur le précurseur cuivre, aluminium, zinc activé thermiquement et mis en forme une imprégnation à sec par une solution de nitrate de lanthane, suivie d'un séchage et d'une activation thermique.

Suivant la méthode décrite pour la préparation du catalyseur A on coprécipite sous forme d'hydroxycarbonate une solution contenant 1 atome-gramme de cuivre, 0,35 atome-gramme d'aluminium et 0,3 atome-gramme de zinc par une solution de carbonate de sodium contenant 2,90 atome-grammes de sodium par litre. Le coprécipité obtenu est ensuite lavé, séché puis calciné comme décrit pour le catalyseur A. Le produit obtenu est enfin mis en forme par pastillage comme décrit pour le catalyseur A.

Les pastilles obtenues sont imprégnées à sec avec un volume d'une solution de nitrate de lanthane (0,05 mole) correspondant au volume poreux du catalyseur (35 $cm^3$); on évapore ensuite le solvant en faisant tourner les pastilles sous balayage d'air chaud, on sèche ensuite pendant 2 heures à 100°C, puis 2 heures à 120°C, le produit est alors activé 2 heures à 350°C sous air.

CATALYSEUR F: (comparaison)

La méthode de préparation du catalyseur F est identique à celle du catalyseur A à l'exception près que l'on ajoute à la solution de carbonate de sodium une certaine quantité de perrhénate de sodium. La solution A est formée par dissolution dans 1,5 l d'eau de 241,6 g de nitrate de cuivre trihydraté (1 at.g Cu), 150 g de nitrate d'aluminium nonahydraté (0,4 at.g Al), 148,75 g de nitrate de zinc hexahydraté (0,5 at.g Zn). La solution A contenant 1,27 at.g de métaux/l est portée à 80°C. La solution B est formée par dissolution dans 1,5 l d'eau de 261,8 g de carbonate disodique (3,29 at.g/l Na) et 2,73 g de perrhénate de sodium (Na Re $O_4$) (0,01 at.g Re). Cette solution B est ensuite portée à 80°C.

CATALYSEUR $F_1$:

La méthode de préparation du catalyseur $F_1$ est identique à celle du catalyseur B aux exceptions près suivantes:

La solution A est formée par dissolution dans 1,5 l d'eau de 241,6 g de nitrate de cuivre trihydraté (1 at.g Cu) de 112,5 g de nitrate d'aluminium nonahydraté (0,3 at.g Al) et de 119 g de nitrate de zinc hexahydraté (0,4 at.g Zn). La solution A (1,133 at.g de métaux /l) est portée à 80°C.

La solution B est formée par dissolution dans 1,5 l d'eau de 234,2 g de carbonate di sodique et de 2,7 g de perrhénate de sodium (Na Re $O_4$) (0,01 at.g Re).

Le précipité $P_1$ cristallisé obtenu suivant le mode opératoire décrit pour la préparation du catalyseur B est lavé 3 fois par 12 l d'eau, puis essoré sur filtre.

Dans un second réacteur on réalise la précipitation, à une température de 80°C, à un pH de 7, de l'hydroxyde de lanthane à partir d'une solution obtenue par dissolution de 43,3 g de nitrate de lanthane dans 0,5 l d'eau (0,1 at.g La) et d'une solution 0,1 molaire d'ammoniaque. Le précipité obtenu est lavé 3 fois par 5 l d'eau, puis essoré sur filtre. Le précipité $P_1$ est mélangé au précipité d'hydroxyde de lanthane par malaxage humide pendant une durée de 30 minutes. Le produit homogène obtenu est alors séché 6 heures à 90°C puis 12 heures à 120°C. Il est ensuite activé sous air à 350°C pendant 4 heures puis mis en forme par pastillage.

CATALYSEUR G: (comparaison)

La méthode de préparation du catalyseur G est identique à celle du catalyseur A à l'exception près que l'on ajoute du nitrate d'argent à la solution des nitrates de cuivre, aluminium et zinc.

La solution A est formée par dissolution dans 1,5 l d'eau de 241,6 g de nitrate de cuivre trihydraté (1 at.g Cu), 112,5 g de nitrate d'aluminium nonahydraté (0,3 at.g Al), 119 g de nitrate de zinc hexahydraté (0,4 at.g Zn), 6,8 g de nitrate d'argent (0,04 at.g Ag). La solution A contenant 1,16 at.g de métaux/l est portée à 80°C.

La solution B est formée par dissolution dans 1,5 l d'eau de 240 g de carbonate disodique (3,02 at.g/l).

CATALYSEUR $G_2$:

Le mode de préparation est identique à celui suivi pour la préparation du catalyseur B à l'exception près que l'on ajoute à la solution des nitrates de cuivre, aluminium et zinc, du nitrate d'argent.

La solution A est formée par dissolution dans 2,5 l d'eau de 241,6 g de nitrate de cuivre trihydraté (1 at.g Cu), 112,5 g de nitrate d'aluminium nonahydraté (0,3 at.g Al), 119 g de nitrate de zinc hexahydraté (0,4 at.g Zn), 6,8 g de nitrate d'argent (0,04 at.g Ag). La solution A contenant 0,7 at.g de métaux/l est portée à 80°C.

La solution B est formée par dissolution dans 1,5 l d'eau de 240 g de carbonate disodique (3,02 at.g Na), elle est portée à 80°C.

Dans un second réacteur, on ajoute simultanément 0,5 l d'une solution C contenant 0,05 mole de nitrate de praséodyme et une solution 0,1 M d'ammoniaque.

Les deux précipités obtenus parfaitement lavés, sont ensuite mélangés par un brassage en suspension dans un réacteur fortement agité.

Après essorage, séchage et activation sous air le catalyseur est mis en forme par pastillage.

CATALYSEUR H:

7

La méthode de préparation est identique à celle du catalyseur B à l'exception près que l'on ajoute du nitrate de zirconyle dihydraté à la solution de nitrate de cérium. Le précipité $P_1$ est formé à partir d'une solution contenant 241,6 g de nitrate de cuivre trihydraté (1 at.g Cu), 75 g de nitrate d'aluminium nonahydraté (0,2 at.g Al), 89,25 g de nitrate de zinc hexahydraté (0,3 at.g Zn) et d'une solution contenant 207 g de carbonate disodique. La précipitation a lieu dans un réacteur à une température de 80°C par addition simultanée des deux solutions à un pH régulé à 6,8.

Le précipité d'hydroxyde de terres rares est formé par addition simultanée dans un réacteur d'une solution contenant 43,43 g de nitrate de cérium hexahydraté (0,1 at.g Ce) et 26,7 g de nitrate de zirconyl dihydraté (0,1 at.g Zr) et d'une solution 0,5 molaire d'ammoniaque. Le pH est maintenu constant à une valeur de 7 et la température est de 80°C.

Les deux précipités après lavages sont mélangés en suspension, un brassage énergique permet d'obtenir une dispersion intime à l'échelle 0,01 à 0,1 micron, avant séchage, activation sous air et mise en forme.

CATALYSEUR $H_1$:

La méthode de préparation est identique à celle du catalyseur B à l'exception près que l'on ajoute un autre métal par imprégnation du précurseur activé sous air et mis en forme. Le précipité $P_2$ est obtenu à partir d'une solution contenant 241,6 g de nitrate de cuivre trihydraté (1 at.g Cu), 56,25 g de nitrate d'aluminium nonahydraté (0,15 at.g Al), 89,25 g de nitrate de zinc hexahydraté (0,3 at.g Zn), et d'une solution contenant 215 g de carbonate disodique.

Le co-précipité d'un métal de terres rares et d'un composé de zirconium est obtenu à partir d'une solution contenant 43,8 g de nitrate de néodyme hexahydraté (0,1 at.g Nd) et 42,95 g de nitrate de zirconium pentahydraté (0,1 at.g Zr), et d'une solution 0,5 molaire d'ammoniaque.

- Après lavage de chaque précipité, mélange en suspension, activation sous air et pastillage, on réalise l'imprégnation des pastilles par une solution contenant 1,55 g de bis acétylacétonate de palladium (0,005 at.g Pd) dans 40 cm³ de toluène. Les pastilles sont ensuite séchées à 90°C dans une étuve ventilée puis activée sous air à 350°C.

Tests des catalyseurs en phase gazeuse.

Tous les catalyseurs sont testés dans une unité pilote fonctionnant en continu et opérant sur 20 ml de catalyseur. Les catalyseurs sont préalablement préréduits dans les conditions suivantes:

-6% hydrogène dans l'azote
- pression atmosphérique
- vitesse spatiale du mélange gazeux égale à 3000 h$^{-1}$
- paliers de réduction
8 h à 160°C
3 h à 190°C
3 h à 210°C
3 h à 240°C

Le déroulement de chaque test est le suivant:
a) Pressurisation de l'unité pilote à 6 MPa par de l'azote.
b) Introduction du mélange $CO + CO_2 + H_2$ sous 6 (MPa) mégapascals à 200°C.
c) Conditions opératoires:

température moyenne du lit de catalyseur     230°C
pression totale     P = 6 MPa
VVH     = 10.000h$^{-1}$

VVH = débit ($H_2 + CO + CO_2$) en litres (TPN)/heure/par litre de catalyseur
composition du gaz de synthèse $\frac{H_2}{2\,CO + 3\,CO_2} = 1,2$

Les résultats de tests sont donnés dans le tableau IV.

Les performances sont définies comme suit:
- productivité massique en méthanol: c'est le nombre de grammes de méthanol obtenus par heure, ramenés au poids (en grammes) de catalyseur chargé.
- sélectivité en méthanol: c'est le rapport du nombre de moles de méthanol formées sur le nombre de moles de ($CO + CO_2$) disparues.

La sélectivité C s'exprime donc par

$$C = 100 \times \frac{\text{nombre de moles de méthanol formées}}{\text{moles } (CO + CO_2)_{\text{entrée}} - \text{moles } (CO + CO_2)_{\text{sortie}}}$$

Exemple 19.

On utilise un réacteur ayant un diamètre de 4 cm et une hauteur utile de 3 mètres contenant 1 l catalyseur ($F_1$).

La réduction du catalyseur est réalisée en phase gazeuse à pression atmosphérique entre 200 et 260°C en injectant simultanément dans le réacteur 3 m³/h d'azote et 200 cm³/h de méthanol.

Après réduction le réacteur est alimenté par une coupe paraffinique $C_{12}$-$C_{18}$ à raison de 270 l/h (vitesse superficielle dans les conditions choisies 7,5 cm/s) et par du gaz de synthèse sous une pression de 7,5 MPa.

Les conditions opératoires sont:

| | |
|---|---|
| pression totale | $P = 7,5\ \text{MPa}$ |
| température | $t = 250°C$ |
| vitesse volumique horaire du gaz | $VVH_g = 15.800\ h^{-1}$ |
| composition du gaz de synthèse | $\dfrac{H_2}{2\,CO + 3\,CO_2} = 1$ |
| vitesse volumique horaire du liquide | $VVH_1 = 270\ h^{-1}$ |

La productivité massique P du catalyseur en méthanol est rapidement stabilisée à 0,5 gramme de méthanol par gramme de catalyseur et par heure.

Exemple 20

Dans un réacteur de type Grignard, fortement agité, de volume 0,5 litre, on introduit 150 cm$^3$ d'une coupe paraffinique $C_{12}$-$C_{18}$ et 10 g de catalyseur B finement broyé (50 à 100μ).

La réduction du catalyseur est réalisée in situ en phase liquide par de l'hydrogène pur à 240°C sous une pression de 7,5 MPa. Après réduction, on substitue l'hydrogène par un mélange $H_2 + CO + CO_2$ de composition telle que: $\dfrac{H_2}{2\,CO + 3\,CO_2} = 1,4$

A 250°C sous une pression de 7,5 MPa la productivité est de 0,6 à 0,7 gramme de méthanol par gramme de catalyseur et par heure.

Exemple 21.

Dans un réacteur tubulaire ayant un diamètre de 0,05 m et une hauteur de 2 m on fait circuler une coupe paraffinique $C_{12}$-$C_{18}$ contenant en suspension (0,2 kg/litre) du catalyseur (H) finement broyé (50-200 μ) et du gaz de synthèse. Une productivité de 1,2 kg de méthanol par kg de catalyseur et par heure a été obtenue dans les conditions opératoires suivantes:

| | |
|---|---|
| -température | $T = 240°C$ |
| - pression totale | $P = 7,5\ \text{MPa}$ |
| - vitesse linéaire de la suspension | $0,2\ \text{cm/s}$ |
| - vitesse volumique horaire du gaz/volume de catalyseur dans le réacteur | $VVH_{gaz} = 10.000\ h^{-1}$ |
| - composition du gaz de synthèse | $\dfrac{H_2}{2\,CO + 3\,CO_2} = 1$ |

Exemple 22.

Dans un réacteur tubulaire, 10 g de catalyseur (C) sont réduits pendant quelques heures à pression atmosphérique par 5% d'hydrogène dans l'azote entre 140 et 260°C. Après réduction, on procède à la décomposition du méthanol. Pour une température de 290°C, une pression de 3,0 MPa et une vitesse spatiale liquide égale à 3h$^{-1}$ plus de 95% du méthanol sont convertis.

TABLEAU I

PHASE RHOMBOEDRIQUE TYPE HYDROXYCARBONATE
MAILLE HEXAGONALE a=0,305 nm C=2,24 nm
GROUPE D'ESPACE R- 3M

| INDICE DE MILLER | d (nm) | i/Lu |
|---|---|---|
| 003 | 0,7557 | 100 |
| 006 | 0,3757 | 75 |
| 101 | 0,2630 | 15 |
| 012 | 0,2585 | 60 |
| 104 | 0,2402 | 10 |
| 015 | 0,2292 | 40 |
| 107 | 0,2053 | 5 |
| 018 | 0,1930 | 30 |
| 1010 | 0,1719 | 15 |
| 0111 | 0,1625 | 10 |
| 110 | 0,1535 | 15 |
| 113 | 0,1507 | 15 |
| 1013 | 0,1454 | 10 |
| 116 | 0,1428 | 10 |
| 0114 | 0,1362 | 5 |

Conditions d'enregistrement: Cu $K_\alpha$ 35 KV 35 mA
Monochromateur arrière (graphite)

9

## TABLEAU II

### COMPOSITION DES CATALYSEURS

| CATALYSEURS (formules) | | RAPPORT ATOMIQUE | | | | | % pds de métal par rapport $\Sigma$pds des métaux | | | | | | TENEUR EN ppm | |
|---|---|---|---|---|---|---|---|---|---|---|---|---|---|---|
| | | Cu/Al | Cu/Zn | Cu/M | Cu/Ln | Cu/Zr | Cu | Al | Zn | Ln | M | Zr | Na | $N_2$ |
| A | $Cu_1Al_{0,4}Zn_{0,3}$ | 2,5 | 3,33 | | | | 67,63 | 11,50 | 20,86 | | | | 132 | - |
| $A_1$ | $Cu_1Al_{0,3}Zn_{0,3}La_{0,1}$ | 3,33 | 3,33 | | 10 | | 60,43 | 7,71 | 18,65 | 13,22 | | | 1500 | 50 |
| $A_2$ | $Cu_1Al_{0,35}Zn_{0,4}La_{0,05}$ | 2,86 | 2,5 | | 20 | | 59,95 | 8,91 | 24,67 | 6,55 | | | 600 | 50 |
| B | $Cu_1Al_{0,15}Zn_{0,3}+Ce_{0,15}$ | 6,66 | 3,33 | | 6,66 | | 58,69 | 3,74 | 18,12 | 19,47 | | | 180 | 550 |
| C | $Cu_1Al_{0,2}Zn_{0,4}+Nd_{0,1}$ | 5 | 2,5 | | 10 | | 58,02 | 4,93 | 23,86 | 13,17 | | | 190 | 650 |
| D | $Cu_1Al_{0,3}Zn_{0,5}+La_{0,1}$ | 3,33 | 2 | | 10 | | 53,75 | 6,86 | 27,64 | 11,76 | | | 100 | - |
| E | $Cu_1Al_{0,25}Zn_{0,4}+La_{0,05}$ | 4 | 2,5 | | 20 | | 61,47 | 6,53 | 25,29 | 6,72 | | | 120 | - |
| $E_1$ | $Cu_1Al_{0,35}Zn_{0,3}+La_{0,05}$ | 2,86 | 3,33 | | 20 | | 63,84 | 9,49 | 19,70 | 6,98 | | | 140 | - |
| F | $Cu_1Al_{0,4}Zn_{0,5}Re_{0,01}$ | 2,5 | 2 | 100 | | | 58,36 | 9,93 | 30,00 | | 1,71 | | 70 | - |
| $F_1$ | $Cu_1Al_{0,3}Zn_{0,4}Re_{0,01}+La_{0,1}$ | 3,33 | 2,5 | 100 | 10 | | 55,96 | 7,14 | 23,02 | 12,24 | 1,64 | | 140 | 700 |
| G | $Cu_1Al_{0,3}Zn_{0,4}Ag_{0,04}$ | 3,33 | 2,5 | 25 | | | 62,23 | 7,94 | 25,60 | | 4,23 | | 130 | 50 |
| $G_1$ | $Cu_1Al_{0,25}Zn_{0,5}Pd_{0,005}+Pr_{0,10}$ | 4 | 2 | 200 | 10 | | 54,07 | 5,68 | 27,80 | 12,0 | 0,47 | | 90 | 600 |
| $G_2$ | $Cu_1Al_{0,3}Zn_{0,4}Ag_{0,04}+Pr_{0,05}$ | 3,33 | 2,5 | 25 | 20 | | 58,22 | 7,42 | 23,95 | 6,43 | 3,96 | | 120 | 250 |
| H | $Cu_1Al_{0,2}Zn_{0,3}+Ce_{0,1}Zr_{0,1}$ | 5 | 3,33 | | 10 | 10 | 56,89 | 4,84 | 17,55 | 12,55 | | 8,17 | 120 | 1050 |
| $H_1$ | $Cu_1Al_{0,15}Zn_{0,3}Zr_{0,1}Nd_{0,1}Pd_{0,005}$ | 6,66 | 3,33 | 200 | 10 | 10 | 57,10 | 3,64 | 17,62 | 12,96 | 0,47 | 8,20 | 190 | 650 |
| $H_2$ | $Cu_1Al_{0,3}Zn_{0,5}Pd_{0,003}+La_{0,05}Zr_{0,05}$ | 3,33 | 2 | 333 | 20 | 20 | 54,71 | 6,97 | 28,14 | 5,98 | 0,27 | 3,93 | 100 | 400 |
| $K_1$ | $Cu_1Al_{0,4}Zn_{0,5}+Pd_{0,005}Pr_{0,1}$ | 2,5 | 2 | 200 | 10 | | 52,23 | 8,88 | 26,86 | 11,59 | 0,44 | | 50 | - |
| $K_2$ | $Cu_1Al_{0,3}Zn_{0,5}+La_{0,1}Pt_{0,002}$ | 3,33 | 2 | 500 | 10 | | 53,58 | 6,82 | 27,56 | 11,71 | 0,33 | | 110 | 800 |

0 155 868

## TABLEAU III

### MODE DE PREPARATION DES CATALYSEURS

| CATALYSEURS | PRECIPITE A | | PRECIPITE B | MELANGE DE A ET B | | | ADDITION D'AUTRES METAUX | | |
| --- | --- | --- | --- | --- | --- | --- | --- | --- | --- |
| | NITRATES | CARBONATE DISSODIQUE SEL DE Na | (par $NH_4OH$ 0,5M) ou 0,1 M | SUSPEN- SION | MALAXAGE HUMIDE | MALAXAGE A SEC | CARBONATE AV. ACTIVATION | OXYDE AP ACTI- VATION | IMPREGNATION APRES MISE EN FORME |
| A | $Cu_1Al_{0,4}Zn_{0,3}$ | | | | | | | | |
| $A_1$ | $Cu_1Al_{0,3}Zn_{0,3}La_{0,1}$ | | | | | | | | |
| $A_2$ | $Cu_1Al_{0,35}Zn_{0,4}La_{0,05}$ | | | | | | | | |
| B | $Cu_1Al_{0,35}Zn_{0,3}$ | | $Ce_{0,15}$ | + | | | | | |
| C | $Cu_1Al_{0,2}Zn_{0,4}$ | | $Nd_{0,1}$ | | + | | | | |
| D | $Cu_1Al_{0,3}Zn_{0,5}$ | | | | | | $La_{0,1}$ | | |
| E | $Cu_1Al_{0,25}Zn_{0,4}$ | | | | | | | $La_{0,05}$ | |
| $E_1$ | $Cu_1Al_{0,35}Zn_{0,3}$ | | | | | | | | $La_{0,05}$ |
| F | $Cu_1Al_{0,4}Zn_{0,5}$ | $0,01\ ReO_4^-$ | | | | | | | |
| $F_1$ | $Cu_1Al_{0,3}Zn_{0,4}$ | $0,01\ ReO_4^-$ | $La_{0,1}$ | | + | | | | |
| G | $Cu_1Al_{0,3}Zn_{0,4}Ag_{0,04}$ | | | | | | | | |
| $G_1$ | $Cu_1Al_{0,25}Zn_{0,5}Pd_{0,005}$ | | $Pr_{0,1}$ | | | + | | | |
| $G_2$ | $Cu_1Al_{0,3}Zn_{0,4}Ag_{0,04}$ | | $Pr_{0,05}$ | + | | | | | |
| H | $Cu_1Al_{0,2}Zn_{0,3}$ | | $Ce_{0,1}\ Zr_{0,1}$ | + | | | | | |
| $H_1$ | $Cu_1Al_{0,15}Zn_{0,3}$ | | $Nd_{0,1}\ Zr_{0,1}$ | | + | | | | 0,005Pd(Acac) |
| $H_2$ | $Cu_1Al_{0,3}Zn_{0,5}Pd_{0,003}$ | | $La_{0,05}Zr_{0,05}$ | + | | | | | |
| $K_1$ | $Cu_1Al_{0,4}Zn_{0,5}$ | | $Pr_{0,1}$ | + | | | | | 0,005 Pd(Cl) |
| $K_2$ | $Cu_1Al_{0,3}Zn_{0,5}$ | | $La_{0,1}$ | + | | | | | 0,002Pt(Acac) |

## TABLEAU IV

### PRODUCTIVITE ET SELECTIVITE DES CATALYSEURS

| Exemples d'utilisation | CATALYSEURS | TEMPERATURE | PRODUCTIVITE (g $CH_3OH$/g cata/h) | | SELECTIVITE "C" en $CH_3OH$ |
|---|---|---|---|---|---|
| | | | 100 h | 1000 h | |
| 1 | A | 230° C | 0,50 | 0,48 | 99,1 |
| 2 | $A_1$ | " | 0,30 | 0,20 | 99,3 |
| 3 | $A_2$ | " | 0,55 | 0,45 | 99,4 |
| 4 | B | " | 0,60 | 0,58 | 99,5 |
| 5 | C | " | 0,62 | 0,60 | 99,4 |
| 6 | D | " | 0,50 | 0,46 | 99,4 |
| 7 | E | " | 0,53 | 0,50 | 99,2 |
| 8 | $E_1$ | " | 0,44 | 0,39 | 98,8 |
| 9 | F | " | 0,55 | 0,51 | 99,0 |
| 10 | $F_1$ | " | 0,62 | 0,60 | 99,6 |
| 11 | G | " | 0,56 | 0,50 | 99,1 |
| 12 | $G_1$ | " | 0,60 | 0,57 | 99,6 |
| 13 | $G_2$ | " | 0,63 | 0,61 | 99,4 |
| 14 | H | " | 0,65 | 0,64 | 99,5 |
| 15 | $H_1$ | " | 0,62 | 0,57 | 99,5 |
| 16 | $H_2$ | " | 0,70 | 0,69 | 99,7 |
| 17 | $K_1$ | " | 0,44 | 0,34 | 98,6 |
| 18 | $K_2$ | " | 0,67 | 0,63 | 99,0 |

Conditions opératoires :

$$T = 230° C$$
$$P = 6 \text{ MPa } (60 \text{ bars})$$
$$VVH = 10.000 \text{ h}^{-1}$$
$$\frac{H_2}{2 \, CO + 3 \, CO_2} = 1,2$$

## Revendications

1. Procédé de préparation d'un catalyseur comprenant au moins quatre éléments essentiels: le cuivre, le zinc, l'aluminium et au moins un métal choisi dans le groupe formé par les terres rares et le zirconium caractérisé en ce qu'il comprend les étapes suivantes:

a) mise en contact d'une solution d'au moins un composé d'ammonium et/ou de métal alcalin choisi dans le groupe formé par les hydrogéno-carbonates, les carbonates et les hydroxydes avec une solution essentiellement homogène contenant sous forme de sels ou de complexes solubles, l'ensemble des métaux cuivre, zinc et aluminium, ladite mise en contact ayant lieu à un pH compris entre 6,3 et 7,3 de manière à former un précipité, cristallisé au moins en partie, des métaux cuivre, zinc et aluminium, ledit précipité étant au moins en partie sous forme d'hydroxycarbonate,

b) lavage à l'eau du précipité obtenu à l'étape a, le produit obtenu ayant une teneur en métaux alcalins réduite à moins de 0,06 % environ en poids par rapport au poids total des métaux du précipité,

c) mise en contact d'une solution contenant un composé d'ammonium choisi dans le groupe formé par l'hydrogéno-carbonate, le carbonate et l'hydroxyde avec une solution d'un sel et/ou d'un complexe soluble d'au moins un métal choisi dans le groupe formé par les terres rares et le zirconium, ladite mise en contact ayant lieu à un pH compris entre 6 et 8, de manière à former un précipité d'au moins un composé de métal du groupe formé par les terres rares et le zirconium,

d) lavage à l'eau du précipité obtenu à 1 étape c de manière à réduire sa teneur en azote à moins de 3 % en poids par rapport aux éléments présents exprimés en poids de métaux,

e) mélange du précipité, cristallisé au moins en partie, obtenu à l'étape b, avec le précipité obtenu à l'étape d de manière à former une dispersion sensiblement homogene,

f) séchage et activation thermique de la dispersion sensiblement homogène obtenue à l'étape e.

2. Procédé de préparation d'un catalyseur selon la revendication 1 caractérisé en ce qu'il comprend les étapes suivantes:

a) mise en contact d'une solution d'au moins un composé d'ammonium et/ou de métal alcalin choisi dans le groupe formé par les hydrogéno-carbonates, les carbonates et les hydroxydes, contenant au plus 4 atomes grammes par litre d'ammonium et/ou de métaux alcalins avec une solution essentiellement homogène, contenant sous forme de sels et/ou de complexes solubles l'ensemble des métaux cuivre, zinc et aluminium à une concentration globale au plus égale à 2 atomes grammes par litre, ladite mise en contact ayant lieu à un pH compris entre 6,3 et 7,3 et à une température d'au moins 60°C, de manière à former un précipité, cristallisé au-moins en partie, des métaux cuivre, zinc et aluminium, ledit précipité étant au moins en partie sous forme d'hydroxycarbonate,

b) lavage à l'eau du précipité cristallisé au moins en partie des métaux cuivre, zinc et aluminium obtenu à l'étape a, le produit obtenu ayant une teneur en métaux alcalins réduite à moins de 0,06 % environ en poids par rapport au poids total des métaux du précipité,

c) mise en contact d'une solution contenant un composé d'ammonium choisi dans le groupe formé par l'hydrogéno-carbonate, le carbonate et l'hydroxyde avec une solution d'un sel et/ou d un complexe soluble d'au moins un métal choisi dans le groupe formé par les terres rares et le zirconium, ladite mise en contact ayant lieu à un pH compris entre 6 et 8 de manière à former un précipité d'au moins un composé de métal du groupe formé par les terres rares et le zirconium,

d) lavage à l'eau du précipité obtenu à l'étape c de manière à réduire sa teneur en azote à moins de 3 % en poids par rapport aux éléments présents exprimés en poids de métaux,

e) mélange du précipité cristallisé au moins en partie, obtenu à l'étape b avec le précipité obtenu à l'étape d de manière à former une dispersion sensiblement homogène,

f) séchage et activation thermique de la dispersion sensiblement homogène obtenue à l'étape e.

3. Procédé de préparation d'un catalyseur selon l'une des revendications 1 ou 2 caractérisé en ce que, au cours de l'étape a, la mise en contact des deux solutions a lieu à un pH compris entre 6,6 et 7,0, le précipité cristallisé au moins en partie obtenu est lavé de manière à obtenir un produit contenant moins de 0,04 % en poids de métaux alcalins par rapport au poids total des métaux du précipité; le précipité d'au moins un métal choisi dans le groupe formé par les terres rares et le zirconium, obtenu à l'étape c, est lavé jusqu'à réduire sa teneur en azote à moins de 1 % en poids par rapport aux éléments présents exprimés en poids de métaux; le mélange des précipités obtenus aux étapes b et d est effectué de manière à obtenir un mélange homogène à l'échelle 0,01-0,1 micron.

4. Procédé selon l'une des revendications 1 à 3 caractérisé en ce que au cours de l'étape a et au cours de l'étape c, les précipités des métaux sont formés à partir de solutions homogènes de nitrates des métaux considérés et à partir de solution de carbonates ou d'hydrogéno-carbonates de métal alcalin pour l'étape a et de solution carbonates ou d'hydrogéno-carbonates d'ammonium pour l'étape c.

5. Procédé selon l'une quelconque des revendications 1 à 4 caractérisé en ce que le catalyseur comprend en outre au moins un métal additionnel M choisi dans le groupe formé par le palladium, l'argent, le rhénium et le platine.

6. Procédé selon la revendication 5 caractérisé en ce que au moins un métal additionnel choisi dans le groupe formé par le palladium, l'argent, et le rhénium est introduit à l'étape a sous forme de composé soluble.

7. Procédé selon l'une quelconque des revendications 1 à 6 caractérisé en ce que au moins un métal additionnel M choisi dans le groupe formé par le palladium, l'argent, le rhénium et le platine est introduit de manière uniforme dans le mélange homogène activé thermiquement obtenu à l'étape f.

8. Procédé selon la revendication 7 caractérisé en ce que au moins un métal additionnel M est introduit à l'aide de sels ou de complexes solubles ne contenant pas de soufre, ni d'halogène dans leurs compositions.

9. Procédé selon l'une quelconque des revendications 1 à 6 caractérisé en ce que, au cours de l'étape a, le temps de résidence du précipité, cristallisé au moins en partie, dans le milieu réactionnel est d'au moins trente minutes.

10. Catalyseur obtenu selon l'une quelconque des revendications 1 à 9, caractérisé en ce que les proportions en poids des métaux des éléments présents rapportés au poids total des métaux sont de:

| Cuivre | 20 - 80 | % |
|---|---|---|
| Zinc | 5 - 50 | % |
| Aluminium | 2 - 30 | % |
| Terres rares et/ou zirconium | 2 - 20 | % |
| Métaux alcalins | 0 - 0,06 | % |
| Métal M | 0 - 10 | % |

11. Catalyseur selon la revendication 10 caractérisé en ce que les proportions en poids des métaux des éléments présents rapportés au poids total des métaux sont de:

| Cuivre | 45 - 70 | % |
|---|---|---|
| Zinc | 15 - 35 | % |
| Aluminium | 4 - 20 | % |
| Terres rares et/ou zirconium | 5 - 18 | % |
| Métaux alcalins | 0 - 0,06 | % |
| Métal M | 0 - 5 | % |

12. Catalyseur selon l'une quelconque des revendications 10 et 11 caractérisé en ce que le métal M est du palladium, du platine ou du rhénium, à la concentration de 0,01 à 1 % en poids et/ou de l'argent à la concentration de 0,5 à 5 % en poids.

13. Utilisation d'un catalyseur obtenu suivant l'une quelconque des revendications 1 à 9, ou d'un catalyseur suivant l'une quelconque des revendications 10 à 12 dans des procédés mettant en jeu une réaction équilibrée des oxydes de carbone ($CO$, $CO_2$) avec l'hydrogène.

14. Utilisation suivant la revendication 13 caractérisée en ce que l'on forme du méthanol à partir d'oxydes de carbone ($CO$, $CO_2$) et d'hydrogène à une température de 200 à 400°C, une pression de 2 à 15 MPa et un rapport molaire $H_2/2 CO + 3 CO_2$ compris entre 0,4 et 10.

15. Utilisation suivant la revendication 13 caractérisée en ce que l'on décompose un alcool de $C_1$ à $C_5$ à une température de 200 à 400°C environ et une pression de 1 à 10 MPa environ pour former un mélange contenant des oxydes de carbone et de l'hydrogène.

**Patentansprüche**

1. Verfahren zur Herstellung eines Katalysators, der zumindest vier wesentliche Elemente enthält: Kupfer, Zink, Aluminium und zumindest ein Metall aus der Gruppe der seltenen Erden und Zirkonium, dadurch gekennzeichnet daß es die folgenden Stufen umfaßt:

a) Inkontaktbringen einer Lösung von zumindest einer Verbindung des Ammoniums und/oder eines Alkalimetalls gewählt aus der Gruppe, die gebildet ist durch die Hydrogencarbonate, die Carbonate und die Hydroxyde, mit einer im wesentlichen homogenen Lösung, die in Form von Salzen oder löslichen Komplexen die Gesamtheit der Metalle Kupfer, Zink und Aluminium enthält, wobei dieses Inkontaktbringen erfolgt bei einem pH zwischen 6,3 und 7,3 und auf solche Weise, daß ein mindestens teilweise kristallisierter Niederschlag der Metalle Kupfer, Zink und Aluminium erfolgt, wobei dieser Niederschlag zumindest teilweise in Form von Hydroxycarbonat erfolgt,

b) Waschen des in Stufe a erhaltenen Niederschlages mit Wasser, wobei das erhaltene Produkt einen Alkaligehalt hat, der auf weniger als etwa 0,06 Gew.-%, bezogen auf das Gesamtgewicht der gefällten Metalle, vermindert ist,

c) Inkontaktbringen einer Lösung, die eine Ammoniumverbindung aus der Gruppe Hydrogencarbonat, Carbonat und Hydroxyd enthält mit einer Lösung eines Salzes und/oder eines löslichen Komplexes von zumindest einem Metall aus der Gruppe seltene Erden und Zirkonium, wobei dieses Inkontaktbringen bei einem pH zwischen 6 und 8 derart erfolgt, daß ein Niederschlag von zumindest einer Verbindung des Metalls der Gruppe seltene Erden und Zirkon gebildet wird,

d) Waschen des in Stufe c erhaltenen Niederschlages mit Wasser derart, daß sein Gehalt an Stickstoff auf unter 3 Gew.-%, bezogen auf die vorhandenen Elemente, ausgedrückt als Gewicht der Metalle, vermindert wird,

14

e) Mischen des zumindest teilweise kristallisierten Niederschlages, der in Stufe b erhalten wurde, mit dem in Stufe d erhaltenen Niederschlag derart, daß eine praktisch homogene Dispersion gebildet wird,

f) Trocknen und thermisches Aktivieren der in Stufe e erhaltenen, praktisch homogenen Dispersion.

2. Verfahren zur Herstellung eines Katalysators nach Anspruch 1, dadurch gekennzeichnet, daß es die folgenden Stufen umfaßt:

a) Inkontaktbringen einer Lösung von zumindest einer Verbindung von Ammonium und/oder Alkalimetall aus der Gruppe Hydrogencarbonate, Carbonate und Hydroxyde, die höchstens 4 Gramm Atom pro Liter Ammonium und/oder Alkalimetall enthält, mit einer im wesentlichen homogenen Lösung, die in Form der Salze und/oder löslicher Komplexe die Gesamtheit der Metalle Kupfer, Zink und Aluminium in einer Gesamtkonzentration von höchstens 2 Gramm Atom pro Liter aufweist, wobei dieses Inkontaktbringen bei einem pH zwischen 6,3 und 7,3 und bei einer Temperatur von wenigstens 60°C derart erfolgt, daß ein mindestens teilweise kristallisierter Niederschlag der Metalle Kupfer, Zink und Aluminium gebildet wird, wobei dieser Niederschlag wenigstens teilweise in Form von Hydroxycarbonat vorliegt,

b) Waschen des zumindest teilweise kristallisierten Niederschlages der Metalle Kupfer, Zink und Aluminium, der in Stufe a erhalten wurde, mit Wasser, wobei das erhaltene Produkt einen Alkaligehalt hat, der auf unter etwa 0,06 Gew.-%, bezogen auf das Gesamtgewicht der gefällten Metalle, vermindert ist,

c) Inkontaktbringen einer Lösung, die eine Verbindung von Ammoniak aus der Gruppe Hydrogencarbonat, Carbonat und Hydroxyd enthält mit einer Lösung eines Salzes und/oder eines löslichen Komplexes von zumindest einem Metall aus der Gruppe seltene Erden und Zirkonium, wobei dieses Inkontaktbringen bei einem pH zwischen 6 und 8 derart erfolgt, daß ein Niederschlag von zumindest einer Verbindung des Metalls der durch seltene Erden und Zirkonium gebildeten Gruppe gebildet wird,

d) Waschen des in Stufe c erhaltenen Niederschlages mit Wasser derart, daß sein Gehalt an Stickstoff auf unter 3 Gew.-%, bezogen auf die vorhandenen Elemente, ausgedrückt als Gewicht der Metalle, vermindert wird,

e) Mischen des zumindest teilweise kristallisierten und in Stufe b erhaltenen Niederschlages mit dem in Stufe d erhaltenen Niederschlag derart, daß eine praktisch homogene Dispersion gebildet wird,

f) Trocknen und thermische Aktivierung der in Stufe c erhaltenen, praktisch homogenen Dispersion.

3. Verfahren zur Herstellung eines Katalysators nach einem der Ansprüche 1 oder 2, dadurch gekennzeichnet, daß im Verlauf der Stufe a das Inkontaktbringen der zwei Lösungen bei einem pH zwischen 6,6 und 7,0 erfolgt, der erhaltene, zumindest teilweise kristallisierte Niederschlag derart gewaschen wird, daß man ein Produkt erhält, das weniger als 0,04 Gew.-% der Alkalimetalle, bezogen auf Gesamtgewicht der Metalle des Niederschlages enthält, der Niederschlag von zumindest einem Metall aus der Gruppe seltene Erden und Zirkonium, der in Stufe c erhalten ist, gewaschen wird, bis sein Gehalt an Stickstoff auf unter 1 Gew.-%, bezogen auf die vorhandenen Elemente, ausgedrückt als Gewicht der Metalle, vermindert ist und die Mischung der in den Stufen b und d erhaltenen Niederschläge derart bewirkt wird, daß man eine Mischung, die in Bereich Var 0,01-0,1 Mikron homogen ist, erhält.

4. Verfahren nach einem der Ansprüche 1 bis 3, dadurch gekennzeichnet, daß im Verlauf der Stufe a und im Verlauf der Stufe c die Niederschläge der Metalle, ausgehend von homogenen Lösungen der Nitrate der fraglichen Metalle und ausgehend von einer Lösung der Carbonate oder Hydrogencarbonate des Alkalimetalls für die Stufe a und einer Ammoniumcarbonat- oder Ammoniumhydrogencarbonatlösung für die Stufe c gebildet werden.

5. Verfahren nach irgendeinem der Ansprüche 1 bis 4, dadurch gekennzeichnet, daß der Katalysator außerdem zumindest ein zusätzliches Metall M enthält, das aus der Gruppe Palladium, Silber, Rhenium und Platin gewählt ist.

6. Verfahren nach Anspruch 5, dadurch gekennzeichnet, daß zumindest ein weiteres Metall aus der Gruppe Palladium, Silber und Rhenium in Stufe a in Form einer löslichen Verbindung eingeführt wird.

7. Verfahren nach irgendeinem der Ansprüche 1 bis 6, dadurch gekennzeichnet, daß zumindest ein weiteres Metall M aus der Gruppe Palladium, Silber, Rhenium und Platin in gleichmäßiger Weise in die in Stufe f erhaltene homogene, thermisch aktivierte Mischung eingeführt wird.

8. Verfahren nach Anspruch 7, dadurch gekennzeichnet, daß zumindest ein weiteres Metall M mit Hilfe der Salze oder der löslichen Komplexe, die weder Schwefel noch Halogen in ihren Verbindungen enthalten, eingeführt wird.

9. Verfahren nach irgendeinem der Ansprüche 1 bis 6, dadurch gekennzeichnet, daß im Verlauf der Stufe a die Verweilzeit des zumindest teilweise kristallisierten Niederschlages im Reaktionsmedium zumindest 30 Minuten beträgt.

10. Katalysator, erhalten nach irgendeinem der Ansprüche 1 bis 9, dadurch gekennzeichnet, daß die Gewichtsanteile der Metalle der vorhanden Elemente, bezogen auf das Gesamtgewicht der Metalle, wie folgt sind:

| | | |
|---|---|---|
| Kupfer | 20 - 80 | % |
| Zink | 5 - 50 | % |
| Aluminium | 2 - 30 | % |
| seltene Erden und/oder Zirkonium | 2 - 20 | % |
| Alkalimetalle | 0 - 0,06 | % |

15

| Metall M | 0 - 10 | % |

11. Katalysator nach Anspruch 10, dadurch gekennzeichnet, daß die Gewichtsanteile der Metalle der vorhandenen Elemente, bezogen auf das Gesamtgewicht der Metalle, wie folgt sind:

| Kupfer | 45 - 70 | % |
| Zink | 15 - 35 | % |
| Aluminium | 4 - 20 | % |
| seltene Erden und/oder Zirkonium | 5 - 18 | % |
| Alkalimetalle | 0 - 0,06 | % |
| Metall M | 0 - 5 | % |

12. Katalysator nach Anspruch 10 oder 11, dadurch gekennzeichnet, daß das Metall M Palladium, Platin oder Rhenium in einer Konzentration von 0,01 bis 1 Gew.-% und/oder Silber in einer Konzentration von 0,5 bis 5 Gew.-% ist.

13. Verwendung eines Katalysators der nach irgendeinem der Ansprüche 1 bis 9 erhalten ist oder eines Katalysators nach einem der Ansprüche 10 bis 12 in Verfahren, bei denen eine ins Gleichgewicht gebrachte Reaktion der Oxide von Kohlenstoff ($CO$, $CO_2$) mit Wasserstoff eingesetzt wird.

14. Verwendung nach Anspruch 13, dadurch gekennzeichnet, daß man Methanol ausgehend von Kohlenoxiden ($CO$, $CO_2$) und Wasserstoff bei einer Temperatur von 200 bis 400°C, einem Druck von 2 bis 15 MPa und einem molaren Verhältnis $H_2/2\,CO + 3\,CO_2$ zwischen 0,4 und 10 bildet.

15. Verwendung nach Anspruch 13, dadurch gekennzeichnet, daß man einen Alkohol mit 1 bis 5 Kohlenstoffatomen bei einer Temperatur von etwa 200 bis 400°C und einem Druck von etwa 1 bis 10 MPa zersetzt und ein Gemisch bildet, das Kohlenoxide und Wasserstoff enthält.

**Claims**

1. A process for manufacturing a catalyst comprising at least, as four essential elements: copper, zinc, aluminum and at least one metal selected from the group consisting of rare earths and zirconium, characterized by the following steps of:

a) contacting a solution of at least one ammonium and/or alkali metal compound, selected from the group consisting of hydrogeno-carbonates, carbonates and hydroxides with an essentially homogeneous solution containing, as salts or soluble complexes, the assembly of copper, zinc and aluminum metals, said contact taking place at a pH ranging from 6,3 to 7,3 so as to form a precipitate, at least partly crystallized, of copper, zinc and aluminum metals, said precipitate being at least partly formed of hydroxycarbonate,

b) washing the precipitate obtained in step a) with water, the obtained product having an alkali metal content reduced to less than about 0,06% by weight with respect to the total metals weight of the precipitate,

c) contacting a solution containing an ammonium compound selected from the group consisting of the hydrogeno-carbonate, carbonate and hydroxide, with a solution of a soluble salt and/or complex of at least one metal selected from the group of the rare earths and zirconium, said contact taking place at a pH from 6 to 8, so as to form a precipitate of at least one compound of metal from the group formed of rare earths and zirconium,

d) washing the precipitate obtained in step c)with water so as to reduce its nitrogen content to less than 3 % by weight with respect to the metals weight,

e) admixing the precipitate, at least partly crystallized, obtained in step b with the precipitate obtained in step d), so as to form a substantially homogeneous dispersion,

f) drying and thermally activating the substantially homogeneous dispersion obtained in step e).

2. A process for manufacturing a catalyst according to claim 1, characterized by the following steps of:

a) contacting a solution of at least one ammonium and/or alkali metal compound, selected from the group of the hydrogeno-carbonates, carbonates and hydroxides,containing a maximum of 4 gram-atoms per liter of ammonium and/or alkali metals, with an essentially homogeneous solution containing the assembly of copper, zinc and aluminum metals, as soluble salts and/or complexes, at a total concentration of at most 2 gram-atoms per liter, said contact taking place at a pH of 6,3 - 7,3 and at a temperature of at least 60°C, so as to form a precipitate, at least partly crystallized, of copper, zinc and aluminum metals, said precipitate being at least partly formed of hydroxycarbonate,

b) washing with water the at least partly crystallized precipitate of copper, zinc and aluminum metals obtained in step a), the resultant product having an alkali metals content reduced to less than about 0,06 % by weight with respect to the total metals weight of the precipitate,

c) contacting a solution containing an ammonium compound selected from the group formed of the hydrogeno-carbonate, carbonate and hydroxide with a solution of a soluble salt and/or complex of at least one metal selected from the group formed of rare earths and zirconium, said contact taking place at a pH from 6 to 8, so as to form a precipitate of at least one metal of the group formed of rare earths and zirconium,

d) washing the precipitate obtained in step c)with water so as to reduce its nitrogen content to less than 3 % by weight with respect to the metals weight,

e) admixing the at least partly crystallized precipitate obtained in step b) with the precipitate obtained in step d)so as to form a substantially homogeneous dispersion,

f) drying and thermally activating the substantially homogeneous dispersion obtained in step e).

3. A process for manufacturing a catalyst according to one of claims 1 or 2, characterized in that, during step a), the two solutions are contacted at a pH from 6,6 to 7,0, the obtained precipitate, at least partly crystallized, is washed so as to obtain a product containing less than 0 04 % by weight of alkali metals with respect to the total metals weight of the precipitate; the precipitate of at least one metal selected from the group formed of rare earths and zirconium, obtained in step c)is washed until its nitrogen content is reduced to less than 1 % by weight with respect to the total metals weight; the precipitates obtained in steps b) and d) being admixed so as to obtain a mixture homogeneous at the scale of 0,01 - 0,1 micron.

4. A process according to one of claims 1 to 3, characterized in that, during step a) and during step c), the metals precipitates are formed from homogeneous solutions of nitrates of the considered metals and from a solution of alkali metal carbonates or hydrogeno-carbonates for step a) and a solution of ammonium carbonates or hydrogeno-carbonates for step c).

5. A process according to any one of claims 1 to 4, characterized in that the catalyst further comprises at least one additional metal M selected from the group formed of palladium, silver, rhenium and platinum.

6. A process according to claim 5, characterized in that at least one additional metal selected from the group formed of palladium, silver and rhenium is introduced in step a), as soluble compound.

7. A process according to any one of claims 1 to 6, characterized in that at least one additional metal M selected from the group formed of palladium, silver, rhenium and platinum is introduced uniformly in the thermally activated homogeneous mixture obtained in step f).

8. A process according to claim 7, characterized in that at least one additional metal M is introduced from salts or complexes containing no sulfur or halogen in their compositions.

9. A process according to any one of claims 1 to 6, characterized in that, during step a), the residence time of the precipitate, at least partly crystallized, in the reaction medium, is at least thirty minutes.

10. A catalyst obtained according to any one of claims 1 to 9, characterized by the following percents by weight of metals, in proportion to the total metals weight:

| | | |
|---|---|---|
| Copper | 20 - 80 | % |
| Zinc | 5 - 50 | % |
| Aluminum | 2 - 30 | % |
| Rare earths and/or zirconium | 2 - 20 | % |
| Alkali metals | 0 - 0,06 | % |
| Metal M | 0 - 10 | % |

11. A catalyst according to claim 10, characterized in that the proportions by weight of metals with respect to the total metals weight are:

| | | |
|---|---|---|
| Copper | 45 - 70 | % |
| Zinc | 15 - 35 | % |
| Aluminum | 4 - 20 | % |
| Rare earths and/or zirconium | 5 - 18 | % |
| Alkali metals | 0 - 0,06 | % |
| Metal M | 0 - 5 | % |

12. Catalyst according to any one of claims 10 and 11, characterized in that metal M is palladium, platinum or rhenium, used at a concentration of 0,01 - 1 % by weight and/or silver at a concentration of 0,5 - 5 % by weight.

13. The use of a catalyst obtained according to any one of claims 1 to 9, or of a catalyst according to any one of claims 10 to 12 in processes involving a balanced reaction of carbon oxides (CO, $CO_2$) with hydrogen.

14. The use according to claim 13, characterized in that methanol is formed from carbon oxides (CO, $CO_2$) and hydrogen at a temperature of 200 - 400°C under a pressure of 2 - 15 MPa and with a molar ratio $H_2$ / 2 CO + 3 $CO_2$ ranging from 0,4 to 10.

15. The use according to claim 13, characterized in that a $C_1$ to $C_5$ alcohol is decomposed at a temperature of about 200-400°C and at a pressure of about 1 - 10 MPa, to form a mixture containing carbon oxides and hydrogen.